# EUROPÄISCHE PATENTSCHRIFT

(11) **EP 0 436 679 B1**
(45) Veröffentlichungstag und Bekanntmachung des Hinweises auf die Patenterteilung: **13.04.1994**
(21) Anmeldenummer: 90908452.7
(22) Anmeldetag: 14.06.1990
(51) Int. Cl.: G01N 27/416, C12M 1/40

(54) **GLUKOSEMESSGERÄT**
GLUCOSE-LEVEL MEASUREMENT DEVICE
APPAREIL DE MESURE DU GLUCOSE

(30) Priorität: 07.07.1989 CH 2535/89
(43) Veröffentlichungstag der Anmeldung: 17.07.1991
(73) Patentinhaber: DISETRONIC HOLDING AG, 3400 Burgdorf (CH)
(72) Erfinder: MICHEL, Peter, CH-3400 Burgdorf (CH); MICHEL, Willy, CH-3400 Burgdorf (CH)
(74) Vertreter: Lusuardi, Werther Giovanni, Dr.
(86) Internationale Anmeldenummer: CH9000146
(87) Internationale Veröffentlichungsnummer: WO9100998

(56) Entgegenhaltungen:
- EP-A- 127 958
- PATENT ABSTRACTS OF JAPAN, vol. 12, no. 203 (P-715)(3050), 11 June 1988 ; & JP-A-635254
- PATENT ABSTRACTS OF JAPAN, vol. 12, no. 112 (P-687)(2959), 9 April 1988 ; & JP-A-62240848
- PATENT ABSTRACTS OF JAPAN, vol. 11, no. 256 (P-607)(2703), 20 August 1987 ; & JP-A-6262260

## Beschreibung

Die Erfindung bezieht sich auf eine Vorrichtung zur Messung und Anzeige der Glukosekonzentration in Blut, gemäss dem Oberbegriff des Anspruchs 1.

Solche Glukosemessgeräte für Diabetiker sind bereits bekannt siehe EP-A-0 127 958 und bestehen in wesentlichen aus den eigentlichen Messgerät (Glucometer) und in darin einsteckbare, auswechselbare Sensoren (Glucosensoren), auf denen das zu bestimmende Blut in kleiner Menge aufgebracht wird. Das dazu notwendige Blut entnimmt sich der Benutzer in herkömmlicher Weise durch Stechen in die Fingerkuppe. Beim Einschieben des Sensors wird dessen Biomembrane mit den Messgerät elektrisch kontaktiert, so dass die durch die unterschiedlichen Glukosegehalte hervorgerufenen Spannungs- und/oder Stromänderungen der aktiven Biomembrane des Sensors an das Messgerät weitergeleitet werden. Der relevante Messbereich der Blutglukose beim Diabetiker liegt zwischen 4 bis 15 mmol/l (≈ 70 - 270 mg/dl). Um auch in klinischen Bereich messen zu können beträgt der Messbereich solcher Geräte typischerweise 3 - 16 mmol/l (≈ 50 - 300 mg/dl).

Die Glukosemessgeräte gemäss den Stand der Technik sind zu kompliziert, schwierig zu bedienen, unzuverlässig und daher auch für den Patienten gefährlich.
Insbesondere hat sich gezeigt, dass die Biomembrane von Glukosesensoren, deren aktiver Bestandteil aus einem instabilen Enzym, beispielsweise Glukoseoxidase, besteht, einerseits eine schwierig reproduzierbare Herstellung aufweist und anderseits verschiedenen alterungsbedingten Veränderung ihrer Eigenschaften unterworfen ist.

Hier will die Erfindung Abhilfe schaffen. Dar Erfindung liegt die Aufgabe zugrunde, ein Glukosemessgerät zu schaffen, mit den es möglich ist alle wesentlichen Einflussgrössen schon bei der Herstellung des Glukosemessgerätes mittels einer einmaligen, für den Patienten unabänderlichen Eichung zu regeln unter gleichzeitiger Berücksichtigung der altersabhängigen Veränderungen der Einflussgrössen.

Die Erfindung löst die gestellte Aufgabe mit einen Glukosemessgerät, welches die Merkmale des Anspruchs 1 aufweist.

In der Praxis wird das erfindungsgemässe Glukosemessgerät in Form eines Anzeigegerätes (Rechner mit Zeitmessgerät) zusammen mit 200 - 300 Sensoren als Gesamtpaket an den Patienten abgegeben. Diese Sensorenanzahl genügt in der Regel für ein halbes bis ein ganzes Jahr. Durch die spezifisch aufeinander abgestimmten Mess- und Auswerte-Charakteristika des Anzeigegerätes und der wegwerfbaren Sensoren, sowie der durch den inneren Zeitmesser des Anzeigegerätes möglichen Anpassung der Auswerteparameter an die altersabhängigen Messparameter der Sensoren wird einerseits eine hohe Genauigkeit des Messergebnisses garantiert und anderseits eine hohe Sicherheit in der Bedienung gewährleistet.

Die durch die Erfindung erreichten Vorteile sind im wesentlichen darin zu sehen, dass dank des erfindungsgemässen Glukosemessgerätes der Patient keine Eichung des Gerätes auf die zu kontaktierenden, wegwerfbaren Sensoren mehr durchführen muss und zwar weder eine Erst-Eichung bei Inbetriebnahme noch eine Nach-Eichung während der Lebensdauer des Gerätes. Dank der automatischen Korrektur sämtlicher wichtiger Parameter und der vorzugsweise vorgesehenen Begrenzung der Lebensdauer der Vorrichtung wird keine Wartung benötigt.

Bei einer bevorzugten Ausführungsform der erfindungsgemässen Vorrichtung wird das Glukosekonzentrationsdiagramm, welches sowohl messstromabhängig, als auch messspannungsabhängig sein kann, mittels einer Ein- oder Mehr-Punkt-Eichung festgelegt. Die Eichung kann dabei sowohl hardwaremässig, z.B. mittels Trimmung in Analogteil (Kalibrieren) oder auch softwaremässig, z.B. mittels einer Tabelle im Rechnungsspeicher erfolgen. Sie gestattet eine auf die mit den Anzeigegerät mitgelieferten, aus einer einzigen Produktionscharge stammenden, wegwerfbaren Sensoren genau abgestimmte, herstellerseitige Eichung, welche patientenseitige Fehler bei dieser heiklen Operation ausschliesst.

Bei einer bevorzugten Ausführungsform der erfindungsgemässen Vorrichtung weisen die einander zugewandten, kuppelbaren Enden von Sensor und Anzeigegerät miteinander korrespondierende Nocken und Nuten auf. Die genaue Form und Anordnung dieser Schlüssel/Schloss-Elemente wird bei der Herstellung der Sensoren und Anzeigegeräte periodisch abgeändert, so dass Sensoren der früheren Produktionschargen - mit einer alterungsbedingt abgeschwächten Reaktionsempfindlichkeit des auf der Biomembrane immobilisierten Enzyms - mit den Anzeigegeräten aus der neuen Produktionscharge nicht mehr verwendet werden können. Damit kann eine weitere patientenseitige Fehler- und Gefahrenquelle weitgehend ausgeschaltet werden.

Alternativ zu dieser Schlüssel/Schloss-Sicherung besteht auch die Möglichkeit die Sensoren mit einem Barcode (Strichcode) zu versehen, der nur von einen für diene Sensoren autorisierten Anzeigerät gelesen werden kann.

Bei einer weiteren bevorzugten Ausführungsform der erfindungsgemässen Vorrichtung ist ein zusätzlicher, vorzugsweise in unmittelbarer Nähe der Biomembrane des Sensors angebrachter Temperatursensor vorgesehen, welcher die Temperaturabhängigkeit des Glukosekonzentrationsdiagrammes berücksichtigt.
Zweckmässigerweise werden dabei die mitgelieferten Sensoren in einer im Anzeigegerät vorgesehenen Schublade (Magazin) aufbewahrt, in welches der Temperatursensor mündet. Dadurch können Temperaturschwankungen, welche ohne weiteres in Bereich zwischen 15 - 25 Grad liegen können, berücksichtigt werden, was zu einer erheblich zuverlässigeren Messung der Glukosewerte führt.

Bei Sensoren gemäss dem Stand der Technik ist die Lagerfähigkeit auf ungefähr ein Jahr beschränkt, deshalb ist bei einer weiteren bevorzugten Ausführungsform der erfindungsgemässen Vorrichtung der Rechner derart vorprogrammiert, dass nach einer vorbestimmten Lebensdauer oder Anzahl durchgeführter Messungen die automatische und unabänderliche Abschaltung und Funktionsuntüchtigkeit der Vorrichtung erfolgt. Diese Funktion kann leicht mit dem vorhandenen inneren Zeitmesser realisiert werden und ist von nicht zu unterschätzender Bedeutung. Die Limitierung der Lebensdauer des Glukosemessgerätes verhindert die Verwendung von zu alten und damit nicht mehr zuverlässig messenden Sensoren. Der Patient wird damit gezwungen sich von Arzt ein neues Gerät mit neuen darauf abgestimmten Sensoren abgeben zu lassen, welches wiederum eine einwandfreie und zuverlässige Ermittlung der Blutglukosewerte sicher stellt. Die Lebensdauer der energieliefernden Batterie sollte zweckmassigerweise auf die derart vorprogrammierte Lebensdauer des Gerätes abgestimmt sein, so dass sich auch ein Batteriewechsel erübrigt.

Zweckmässigerweise wird bei der erfindungsgemässen Vorrichtung der Rechner derart vorprogrammiert, dass die vom Zeitmessgerät beeinflusste, automatische und unabänderliche Korrektur des Glukosekonzentrationsdiagrammes, welche die zeitabhängigen Eigenschaften der wegwerfbaren Sensoren berücksichtigt, mittels eines chargenabhängigen Reaktionsempfindlichkeitsdiagrammes erfolgt. Damit ist es möglich die mit zunehmender Alterung gesetzmässig abnehmende Reaktionsempfindlichkeit des auf der Biomembrane des Sensors immobilisierten Enzyms, beispielweise Glukoseoxidase, zu berücksichtigen. Die Aktivitätsminderung gehorcht in einer ersten Näherung einer abnehmenden e-Funktion, welche in Form eines Programmes oder einer Tabelle im Rechner gespeichert werden kann.

Bei einer weiteren bevorzugten Ausführungsform der erfindungsgemässen Vorrichtung ist vorgesehen, dass der Rechner über einen Speicher verfügt, mit welchen die gemessenen Blutglukosewerte für spätere Auswertungen gespeichert werden können. Dies ermöglicht beispielsweise das Überspielen der Glukosewerte einer ganzen Messperiode (30 - 50 Messwerte) in ein externes Gerät (z.B. Personal Computer), wo sie weiterverarbeitet und ausgewertet werden können um die Therapie zu unterstützen.

Die interne Uhr ist derart konzipiert, dass sie der Patient nicht selbst stellen kann. Bei Bedarf kann sie durch den Arzt extern reguliert werden. Die Zeit ist aber nur in Zusammenhang mit den gespeicherten Messwerten für den Blutglukosegehalt interessant, so dass die Uhr-Justierung zweckmässigerweise beim Auslesen der Werte erfolgen kann.

Ein Ausführungsbeispiel der Erfindung, welches zugleich das Funktionsprinzip erläutert, ist in der Zeichnung dargestellt und wird im folgenden näher beschrieben.
Fig. 1 stellt eine schematische Gesamtansicht des Messgerätes mit einem eingesteckten Messtreifen dar;
Fig. 2 stellt eine Seitenansicht des Messgerätes mit Abdeckkappe dar;
Fig. 3 stellt eine Aufsicht auf einen Sensor dar;
Fig. 4 stellt eine Frontansicht des Sensors dar;
Fig. 5 stellt eine Frontansicht des Anzeigegerätes dar;
Fig. 6 stellt ein Diagramm Glukosekonzentration/Messstrom mit chargenmässig unterschiedlichen Kurven und ihre Alterungsabhängigkeit dar; und
Fig. 7 stellt ein Rekationsempfindlichkeitsdiagramm dar.

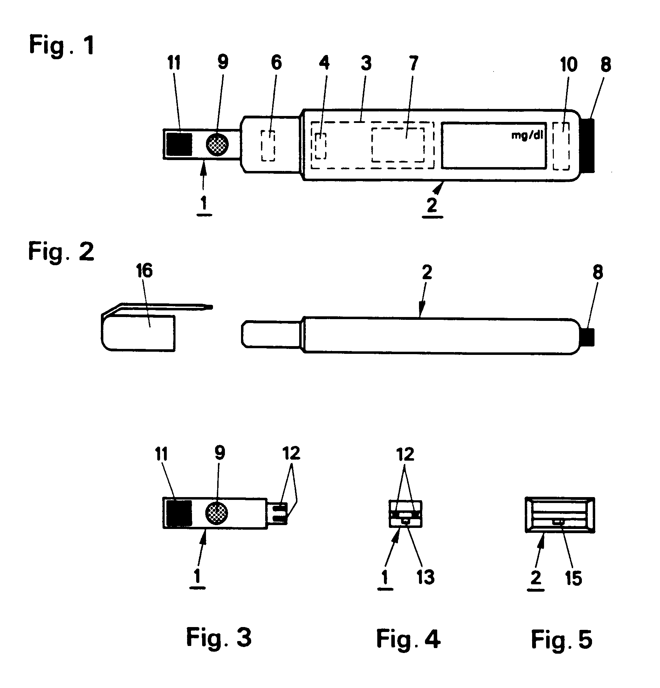

Das in Fig. 1 im Messzustand dargestellte Anzeigegerät 2 mit eingesteckten Sensor 1 besteht in wesentlichen aus einem Rechner 3 mit einer inneren Uhr 4 und einem Speicher 7, einen Temperatursensor 6, einer Batterie 10 und einem sämtliche Funktionen des Gerätes in Gang setzenden Druckknopf 8.

Das für den Einsteck-Kontakt mit dem Sensor 1 bestimmte Ende des Gerätes 2 wird im Ruhezustand, wie in Fig. 2 dargestellt, mit dem Aufsteck-Clip 16 verschlossen, welcher gleichzeitig als Magazin für die noch nicht benutzen Sensoren 1 dient. Dies hat den Vorteil, dass der Temperatursensor 6 einen der tatsächlichen Temperatur des Sensors 1 möglichst nahekommenden Wert misst und an den Rechner 3 weiterleitet.

Der Sensor 1, welcher in den Figuren 1, 3 und 4 im Detail dargestellt ist, besteht aus einem länglichen Streifen aus Kunststoff, der an einem Ende die elektrischen Kontakte 12 zur Verbindung mit den Anzeigegerät 2 aufweist und am anderen Ende eine Grifffläche 11 für dessen Manipulation (Ein- und Ausstecken) besitzt. Im Zentrum des Sensors 1 ist die Biomembrane 9 angeordnet, auf welche der zu messende Bluttropfen aufzubringen ist. Die Biomembrane 9 besteht aus einer amperometrischen Elektrode mit einer Enzymmembrane, die aus Glukoseoxidase besteht. Am steckerseitigen Ende des Sensors 1 ist zwischen den elektrischen Kontakten 12 ein Nocken 13 angebracht, der in eine entsprechende Nut 15 des steckerseitigen Endes des Anzeigegerätes 2 passt (Fig. 5).
Die genaue Form und Anordnung dieser Schlüssel/Schloss-Elemente 13,15 wird bei der Herstellung der Sensoren 1 und Anzeigegeräte 2 zweckmässigerweise alle 6 bis 12 Monate abgeändert, so dass Sensoren 1 der früheren Produktionschargen - mit einer alterungsbedingt abgeschwächten Reaktionsempfindlichkeit der Glukoseoxidase - mit den Anzeigegeräten 2 aus der neuen Produktionscharge nicht mehr verwendet werden können.
Alternativ zu dieser Schlüssel/Schloss-Sicherung 13,15 besteht auch die Möglichkeit die Sensoren 1 mit einem Barcode (Strichcode) zu versehen, der nur von einem für diese Sensoren 1 autorisierten Anzeigerät 2 gelesen werden kann.

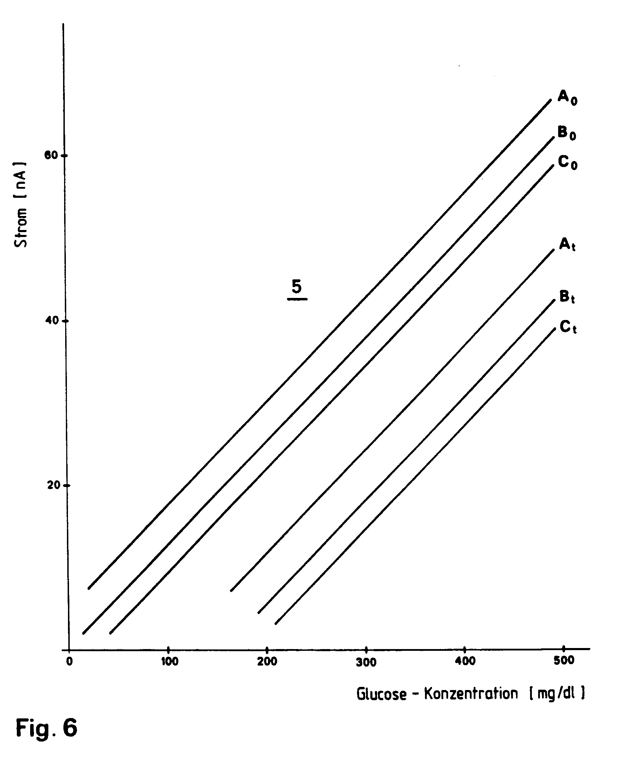

Vor der Auslieferung des Anzeigegerätes 2 an den Patienten, bzw. den Handel wird dieses von Hersteller auf die gleichzeitig mitzuliefernden Sensoren 1 geeicht, d.h. die Nulleinstellung des Rechners 3 wird mit dem Herstellungsdatum der wegwerfbaren Sensoren 1 in für den Patienten unabänderlicher Weise korreliert. Wie in Fig. 6 dargestellt besteht das Glukosekonzentrationsdiagrammes 5 aus einer Vielzahl von chargenabhängigen Kurven A₀, B₀, C₀ (mit Gültigkeit für den Zeitpunkt t = 0, d.h. im Zeitpunkt der Herstellung der Sensoren 1). Je nach Herstellungscharge zeigt das auf der Biomembrane 9 des Sensors 1 immobilisierte Enzym (z.B. Glukoseoxidase) eine mehr oder weniger grosse Aktivität (Charge A : Eichkurve A₀ / Charge B: Eichkurve B₀ / Charge C: Eichkurve C₀). In der Regel ist die chargenabhängige Glukoseeichkurve A₀, B₀, C₀ linear und weist in erster Näherung eine konstante Steigung auf. Für den Eichvorgang ist somit in der Regel eine Ein-Punkt-Eichung genügend. Für kompliziertere Eichkurven, wie sie sich bei Verwendung anderer Enzyme ergeben können, wäre eine Zwei- oder Mehr-Punkt-Eichung nötig.

Mit dieser Erst- oder Nulleichung besitzt der Speicher 7 des Rechners das zum Zeitpunkt t = 0 der Herstellung einer bestimmten Charge von Sensoren 1 gültige Glukosekonzentrationsdiagramm 5, z.B. C₀ (entsprechend den mitgelieferten Sensoren der Charge C) für eine gewählte, mittlere Standardtemperatur.

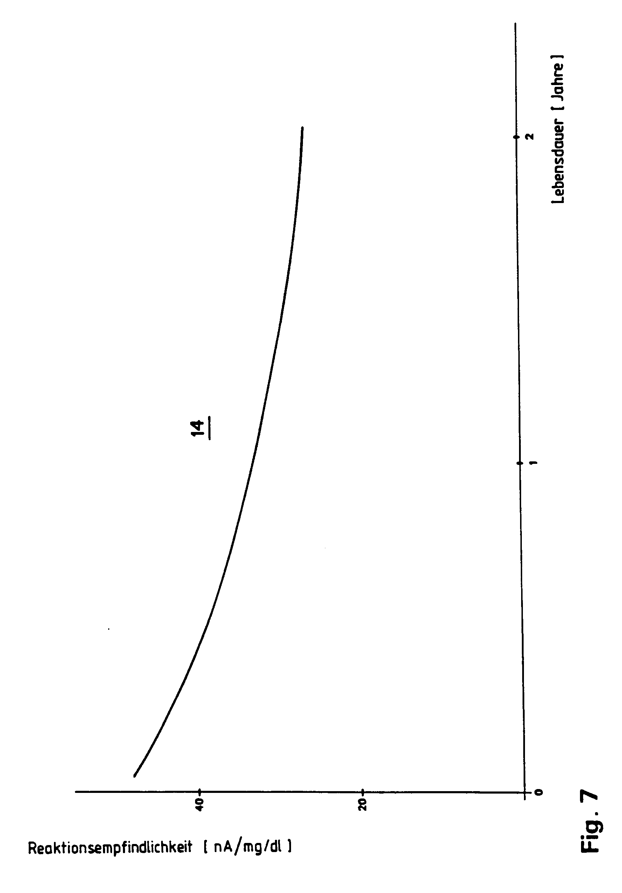

Bedingt durch die Alterung des verwendeten Enzyms ergibt sich, wie in Fig. 7 dargestellt, eine mit fortschreitendem Alter abnehmende Aktivität des Enzyms, was sich im Glukosekonzentrationsdiagrammes 5 (Fig. 6) durch eine Verschiebung der Eichkurven A₀, B₀, C₀ nach unten (Aₜ, Bₜ, Cₜ) manifestiert. Mittels der inneren Uhr 4 und der im Speicher 7 vorhandenen Glukosekonzentrationsdiagramme 5 und Enzymreaktionsempfindlichkeitsdiagramme 14 kann der Rechner 3 eine vom Patienten unbeeinflusste, automatische Bestimmung der jeweils gültigen Glukoseeichkurve, z.B. Cₜ (für Sensoren der Charge C vom Alter t) durchführen, welche die zeitabhängigen Eigenschaften der Biomembrane 9 der wegwerfbaren Sensoren 1 berücksichtigt.

Sämtliche in Fig. 6 dargestellten Eichkurven sind für eine bestimmte mittlere Standardttemperatur berechnet. Da die Reaktionsempfindlichkeit des auf der Biomembrane 9 immobilisierten Enzyms von der Temperatur abhängig ist wird zweckmässigerweise eine Temperaturkorrektur durchgeführt. Der Temperaturkoeffizient variiert für die einzelnen Enzyme und beträgt für die bevorzugt verwendete Glukoseoxidase ca. 5%/°C (bei einer Konzentration von 20 mmol/l), wobei die Reaktionsempfindlichkeit mit zunehmender Temperatur ebenfalls steigt, d.h. sich gleichsinnig verändert. Neben der für eine gewählte Standardtemperatur gültigen Kurve Cₜ (für Sensoren der Charge C des Alters t) sind somit im Speicher 7 des Rechners 3 davon abgeleitete, von der vom Temperatursensor 6 tatsächlich gemessenen Temperatur abhängige Kurven gespeichert.

## Patentansprüche

1. Vorrichtung zur Messung und Anzeige der Glukosekonzentration im Blut mittels wegwerfbarer Sensoren (1) mit einer Biomembrane (9) und einen eichbaren auf der Basis des Glukosekonzentrationsdiagrammes (5) arbeitenden, die Messergebnisse der Sensoren (1) auswertenden Anzeigegerätes (2), wobei
A) das Anzeigegerät (2) einen Rechner (3) mit einem Zeitmessgerät (4) aufweist, dessen Nulleinstellung mit dem Herstellungsdatum der wegwerfbaren Sensoren (1) in unabänderlicher Weise korrelierbar ist;
B) eine unabänderliche Eichung des Glukosekonzentrationsdiagrammes (5) vorgesehen ist, welche den Eigenschaften der Biomembrane (9) der wegwerfbaren Sensoren (1) zum Zeitpunkt ihrer Herstellung entspricht;
C) mindestens eine vom Zeitmessgerät (4) beeinflusste, automatische und unabänderliche Korrektur des Glukosekonzentrationsdiagrammes (5) vorgesehen ist, welche die zeitabhängigen Eigenschaften der Biomembrane (9) der wegwerfbaren Sensoren (1) berücksichtigt.

2. Vorrichtung nach Anspruch 1, dadurch gekennzeichnet, dass die Eichung des Glukosekonzentrationsdiagrammes (5) eine Mehrfach-Eichung, vorzugsweise eine Zweipunkt-Eichung ist.

3. Vorrichtung nach Anspruch 1 oder 2, dadurch gekennzeichnet, dass ein zusätzlicher, vorzugsweise in unmittelbarer Nähe der Sensoren angebrachter Temperatursensor (6) vorgesehen ist, welcher die Temperaturabhängigkeit des Glukosekonzentrationsdiagrammes (5) berücksichtigt.

4. Vorrichtung nach einem der Ansprüche 1 bis 3, dadurch gekennzeichnet, dass der Rechner (3) derart vorprogrammiert ist, dass nach einer vorbestimmten Lebensdauer oder Anzahl von Messungen die automatische und unabänderliche Abschaltung und Funktionsuntüchtigkeit der Vorrichtung erfolgt.

5. Vorrichtung nach einem der Ansprüche 1 bis 4, dadurch gekennzeichnet, dass der Rechner (3) über einen Speicher (7) verfügt, mit welchem die gemessenen Blutglukosewerte für spätere Auswertungen gespeichert werden können.

6. Vorrichtung nach einem der Ansprüche 1 bis 4, dadurch gekennzeichnet, dass sämtliche Funktionen der Vorrichtung mittels eines einzigen Druckknopfes (8), vorzugsweise mit einer taktilen Rückkoppelung ausgelöst werden.

7. Vorrichtung nach einen der Ansprüche 1 bis 6, dadurch gekennzeichnet, dass der Sensor (1) eine amperometrische Elektrode mit einer Enzymmembrane, vorzugsweise Glukoseoxidase besteht.

8. Vorrichtung nach einem der Ansprüche 1 bis 7, dadurch gekennzeichnet, dass das Glukosekonzentrationsdiagramm (5) in Abhängigkeit von Messstrom dargestellt ist.

9. Vorrichtung nach einen der Ansprüche 1 bis 8, dadurch gekennzeichnet, dass der Rechner (3) derart vorprogrammiert ist, dass die vom Zeitmessgerät (4) beeinflusste, automatische und unabänderliche Korrektur des Glukosekonzentrationsdiagrammes (5), welche die zeitabhängigen Eigenschaften der wegwerfbaren Sensoren (1) berücksichtigt, mittels eines chargenabhängigen Reaktionsempfindlichkeitsdiagrammes (14) erfolgt.

10. Vorrichtung nach einem der Ansprüche 1 bis 9, dadurch gekennzeichnet, die einander zugewandten, kuppelbaren Enden von Sensor (1) und Anzeigegerät (2) miteinander korrespondierende Schlüssel/Schloss-Elemente, vorzugsweise in Form von Nocken (13) und Nuten (15) aufweisen.

## Claims

1. Device for measuring and displaying the glucose concentration in the blood by means of disposable sensors (1) comprising a biomembrane (9) and a display device (2) which can be calibrated and works on the basis of the glucose concentration diagram (5) and evaluates the measurement results of the sensors (1), whereby
A) the display device (2) comprises a computer (3) with a time measuring device (4), whose zero setting can be correlated with the date of manufacture of the disposable sensors (1) in an irreversible manner;
B) an irreversible calibration of the glucose concentration diagram (5) is provided which corresponds to the characteristics of the biomembrane (9) of the disposable sensors (1) at the time of their manufacture;
C) at least one automatic and irreversible correction of the glucose concentration diagram (5) which is influenced by the time measuring device (4) is provided which takes into account the time-dependent characteristics of the biomembrane (9) of the disposable sensors (1).

2. Device according to claim 1, characterized in that the calibration of the glucose concentration diagram (5) is a multiple calibration, preferably a two-point calibration.

3. Device according to claim 1 or 2, characterized in that an additional temperature sensor (6) is provided, which is preferably arranged in the immediate vicinity of the sensors and takes into account the dependence of the glucose concentration diagram (5) on temperature.

4. Device according to one of the claims 1 to 3, characterized in that the computer (3) is pre-programmed in such a way that the device is automatically and irreversibly switched off and made inoperative after a predetermined service life or number of measurements.

5. Device according to one of the claims 1 to 4, characterized in that the computer (3) comprises a memory (7) by means of which the measured blood glucose values can be stored for subsequent evaluations.

6. Device according to one of the claims 1 to 4, characterized in that all functions of the device are triggered by means of a single push button (8), preferably with a tactile reaction.

7. Device according to one of the claims 1 to 6, characterized in that the sensor (1) comprises an amperometric electrode with an enzyme membrane, preferably glucose oxidase.

8. Device according to one of the claims 1 to 7, characterized in that the glucose concentration diagram (5) is shown as a function of the measurement current.

9. Device according to one of the claims 1 to 8, characterized in that the computer (3) is pre-programmed in such a way that the automatic and irreversible correction of the glucose concentration diagram (5), which is influenced by the time measuring device (4) and takes into account the time-dependent characteristics of the disposable sensors (1), is effected by means of a batch-dependent reaction sensitivity diagram (14).

10. Device according to one of the claims 1 to 9, characterized in that the ends of the sensor (1) and display device (2), which face one another and can be coupled, comprise key/lock elements which correspond with one another, preferably in the form of cams (13) and grooves (15).

## Revendications

1. Dispositif de mesure et d'affichage de la concentration en glucose dans le sang au moyen de capteurs jetables (1) comprenant une biomembrane (9) et un dispositif d'affichage (2) qui peut être calibré et qui opère sur la base du diagramme de concentration en glucose (5) et évalue les résultats des mesures des capteurs (1), dans lequel
A) le dispositif d'affichage (2) comprend un calculateur (3) avec un dispositif de mesure du temps (4), dont la mise à zéro peut être mise en corrélation de manière irréversible avec la date de fabrication des capteurs jetables (1);
B) un calibrage irréversible du diagramme de concentration en glucose (5) est fourni, qui correspond aux caractéristiques de la biomembrane (9) des capteurs jetables (1) au moment de leur fabrication;
C) au moins une correction automatique et irréversible du diagramme de concentration en glucose (5), qui est influencée par le dispositif de mesure du temps (4), est fournie et tient compte des caractéristiques dépendantes du temps de la biomembrane (9) des capteurs jetables (1).

2. Dispositif selon la revendication 1, caractérisé en ce que le calibrage du diagramme de concentration en glucose (5) est un calibrage multiple, de préférence un calibrage à deux points.

3. Dispositif selon la revendication 1 ou 2, caractérisé en ce qu'un capteur supplémentaire de température (6) est fourni, qui est disposé de préférence au voisinage immédiat des capteurs et tient compte de la dépendance du diagramme de la concentration en glucose (5) vis-à-vis de la température.

4. Dispositif selon l'une des revendications 1 à 3, caractérisé en ce que le calculateur (3) est préprogrammé de telle manière que le dispositif est automatiquement déconnecté et rendu inopérant de manière irréversible après une durée de service prédéterminée ou un nombre de mesures prédéterminé.

5. Dispositif selon l'une des revendications 1 à 4, caractérisé en ce que le calculateur (3) comprend une mémoire (7) au moyen de laquelle les valeurs mesurées du glucose dans le sang peuvent être mises en mémoire pour des évaluations postérieures.

6. Dispositif selon une des revendications 1 à 4, caractérisé en ce que toutes les fonctions du dispositif sont déclenchées au moyen d'un simple boutonpoussoir (8), de préférence à réaction tactile.

7. Dispositif selon une des revendications 1 à 6, caractérisé en ce que le capteur (1) comprend une électrode ampérométrique avec une membrane à enzyme, de préférence de l'oxydase de glucose.

8. Dispositif selon une des revendications 1 à 7, caractérisé en ce que le diagramme de concentration en glucose (5) est présenté sous forme d'une fonction du courant de mesure.

9. Dispositif selon une des revendications 1 à 8, caractérisé en ce que le calculateur (3) est préprogrammé de telle manière que la correction automatique et irréversible du diagramme de concentration en glucose (5), qui est influencée par le dispositif de mesure du temps (4) et tient compte des caractéristiques dépendantes du temps des capteurs jetables (1), soit effectuée au moyen d'un diagramme de sensibilité de réaction par lots (14).

10. Dispositif selon une des revendications 1 à 9, caractérisé en ce que les extrémités du capteur (1) et du dispositif d'affichage (2), qui se font face et peuvent être couplées, comprennent des éléments de verrouillage et de déverrouillage qui correspondent mutuellement, de préférence sous la forme de cames (13) et de sillons (15).
